# EUROPEAN PATENT APPLICATION

(11) **EP 4 219 698 A1**
(43) Date of publication of application: **02.08.2023**
(21) Application number: 22153506.5
(22) Date of filing: 26.01.2022
(51) Int. Cl.: C12N 9/14, C12N 9/22, C12N 9/99, A61P 35/02

(54) **PEPTIDE AND USE THEREOF IN THE PREVENTION OF THERAPY-RELATED LEUKEMIA**

(71) Applicant: Universität Ulm, 89081 Ulm (DE)
(72) Inventor: Wiesmüller, Elisabeth, 89231 Neu-Ulm (DE); Sanchez Garcia, Elsa, 44797 Bochum (DE); Ruiz Blanco, Yasser Bruno, 45128 Essen (DE); Wille, Julia, 89073 ulm (DE); Gole, Boris, 1241 Kamnik (SI); Palmer, Anja, 73340 Amstetten-Bräunisheim (DE); Gebhardt, J. Christof M., 89075 Ulm (DE)
(74) Representative: Michalski Hüttermann & Partner Patentanwälte mbB

(57) **Abstract**

The present invention relates to a peptide comprising an amino acid sequence EX₁EAX₂SGSS according to SEQ ID No. 1, or a pharmaceutically acceptable salt thereof, wherein X₁ and X₂ independently represent a linker which links the adjoining amino acids or an amino acid deletion, and wherein the peptide has an overall length in the range of from 7 to 30 amino acids. The present invention further relates to a pharmaceutical composition comprising the peptide, and the peptide for use in the preventive treatment of acute lymphoblastic leukemia, myelodysplastic syndrome or acute myeloid leukemia, particularly therapy-related acute lymphoblastic leukemia, therapy-related myelodysplastic syndrome or therapy-related acute myeloid leukemia.

## Description

Chromosomal rearrangements in the *Mixed Lineage Leukemia* gene (*MLL*/*KMT2A*) have been observed frequently in leukemia cells in infants and after chemotherapy or radiotherapy. Leukemias associated with rearrangements of *MLL* are characterized by a poor prognosis in terms of overall survival and event-free survival. Acute therapy-associated leukemias account for 10-20% of all acute leukemias. The relevance of research on therapy-induced, so-called secondary, leukemias is underlined by the fact that the number of surviving cancer patients increases by 3% per year and thus an increased risk for therapy-induced carcinomas is to be expected.

Endonuclease G (EndoG) has been identified as a key molecule in these leukemia-causing processes. EndoG originally is known as a nuclease that cleaves the genome at the onset of active cell death. Recently it has become clear that genome cleavage by apoptotic nucleases can also proceed sublethally and, in turn, trigger cancer-causing genome alterations. After replication stress, which can be triggered e.g. by chemotherapy or radiotherapy, sublethal breakage occurs followed by rearrangements taking place in a 7.3kb breakpoint region in *MLL,* specifically in a 0.4 kb hotspot for therapy-induced rearrangements (MLLbcr). For these processes EndoG was identified as a trigger. Consequently, inhibitors of EndoG are useful for protecting normal cells when DNA-damaging and cell death-inducing chemotherapeutics are used to promote cell death in cancer cells.

WO 2014/176351 A1 describes small molecule EndoG inhibitors that suppress the catalytic activity of EndoG and, to a lesser extent, the activity of other nucleases in the cell, and describes their cytoprotective effect on tumor cell lines. The described nonspecific effect on nuclease activity may prevent the cytotoxic effect of EndoG during apoptosis, which, however, may also undesirably mediate chemotherapy resistance. Targeted interference, which selectively blocks only certain molecular interactions, is difficult to achieve with conventional chemical substances such as the so-called small molecules.

Therefore, the objective underlying the present invention was to provide improved means for EndoG inhibition, in particular, by selectively blocking EndoG nuclease activity.

The problem is solved by a peptide comprising an amino acid sequence EX₁EAX₂SGSS according to SEQ ID No. 1, or a pharmaceutically acceptable salt thereof, wherein X₁ and X₂ independently represent a linker which links the adjoining amino acids or an amino acid deletion, and wherein the peptide has an overall length in the range of from 7 to 30 amino acids.

Surprisingly, peptides were found that inhibit EndoG with high specificity, but were shown to not inhibit the cytotoxic effects of chemotherapeutic agents. The peptides provide very high specificity for EndoG and inhibit its nuclease activity that triggers MLLbcr rearrangements during replication stress. This allows the reduction of a severe side effect of chemo- and radiotherapies, namely the development of secondary tumors, especially leukemias, by rearrangements at the MLLbcr. Thus, secondary leukemias can be prevented or at least be reduced by preventive combination therapy with the peptides. Without being bound to a specific theory, it is assumed that the peptides provide a protective effect against genome rearrangements that cause secondary leukemia by reducing the fragility and frequency of rearrangements in *MLL* within a 0.4 kb short region, where rearrangements are frequently detected in patients with secondary leukemia as well as in infants with acute lymphoblastic leukemia (ALL).

It was found that the C-terminal domain of the human protein Ku80 reduces MLLbcr rearrangements in leukemia cells after treatment with chemotherapeutic agent doxorubicin. It was further found that peptides EIVVQDGITLITKEEASGSSVTAEEAKK (SEQ ID No. 3) and ITKEEASGSSVTAEEAKKFLA (SEQ ID No. 4) were able to reduce MLLbcr rearrangements in leukemia cells after doxorubicin treatment without exhibiting toxic effects. Without being bound to a specific theory, it is assumed that the motif EX₁EAX₂SGSS according to SEQ ID No. 1 is relevant for binding of the peptides to EndoG and specific EndoG inhibition.

Advantageously, the peptides are able to provide anti-mutagenic and anti-carcinogenic properties without simultaneously affecting the underlying DNA double-strand break repair activities in the cell. No changes in survival or apoptosis of doxorubicin-treated cells after treatment with the peptides were observed. Thus, parallel preventive treatment with the peptides can reduce the side effect of leukemia-causing genome rearrangements, but will not impair the killing of tumor cells intended by chemotherapy.

As used herein, the term "peptide" is understood to refer to synthetic or non-synthetic peptide compounds as well as purified and modified fragments of natural proteins, native forms or recombinant peptides. Likewise, the term "peptide" in the sense of the present invention includes pharmacologically acceptable salts. Further, the peptide may comprise a detectable label, such as a radionuclide, a fluorophore or biotin. Further, the term "peptide" in the sense of the present invention includes conjugates of the corresponding peptide. Preferred conjugates include, for example, sugar or polyethylene glycol conjugates, biotinylated, radioactive or fluorescence-labeled peptides.

As used herein, the term "EndoG" refers to Endonuclease G, a nuclear DNA-coded mitochondrial nuclease encoded by the *ENDOG* gene that translocates to the nucleus and degrades DNA during apoptosis.

The motif EX₁EAX₂SGSS according to SEQ ID No. 1 is assumed to be important for binding to EndoG with high specificity. Without being bound to a specific theory, it is assumed that the peptide's inhibiting properties for EndoG nuclease activity depend on the presence of this binding motif.

The motif EX₁EAX₂SGSS comprises invariable amino acids and two variable members X₁ and X₂ that may be present in the motif or may not be present. X₁ and X₂ accordingly independently represent linkers which link the adjoining amino acids or an amino acid deletion. It was found that a sequence of amino acids EEASGSS (SEQ ID No. 2) is invariable, but the starting glutamic acid residues may be separated by a linker. Also, alanine and serine may be spaced apart by a linker. The linker may be selected from a polyethylene glycol linker, an alkyl linker or one or more amino acids. In embodiments, the linker may be a polyethylene glycol (PEG) linker. The amino acids may be conjugated to the polyethylene glycol molecules via covalent linkage, non-covalent linkage, or both. The PEG moiety conjugated to the motif can be linear or branched. Polyethylene glycol is a biologically inert, non-immunogenic compound, and is non-toxic and highly flexible. In other embodiments the linker may be a linear or branched, saturated or unsaturated alkyl group. In other embodiments the linker may be a peptide linker. A peptide linker preferably comprises amino acids selected from the group of glutamic acid, serine, alanine, and glycine.

In embodiments, X₁ and X₂ independently represent one or two contiguous amino acids selected from the group of glutamic acid, serine, alanine, and glycine or an amino acid deletion. In embodiments, X₁ and X₂ independently may represent one or two amino acids selected from glutamic acid, serine, and alanine or X₁ and X₂ independently may represent one or two amino acids selected from serine, alanine, and glycine. In embodiments, X₁ represents one or two amino acids selected from the group of glutamic acid, serine, alanine, and glycine and X₂ is an amino acid deletion according to EX₁EASGSS (SEQ ID No. 7) such that the starting glutamic acid residues are separated by a linker.

In preferred embodiments, the amino acid sequence is EEASGSS according to SEQ ID No. 2. It was found in silico that this minimal motif was relevant for binding to EndoG with high specificity. Without being bound to a specific theory, it is assumed that the motif of SEQ ID No. 2 is crucial for the inhibiting properties on EndoG nuclease activity by blocking specific protein-protein interactions of human EndoG in human cells and thus reducing the abundance of MLLbcr rearrangements.

The peptide thus has a minimal length of 7 amino acids and may have a length of up to 30 amino acids. In embodiments, the peptide has an overall length in the range of from 20 to 28 amino acids.

In preferred embodiments, the peptide comprises or consists of the amino acid sequence EIVVQDGITLITKEEASGSSVTAEEAKK according to SEQ ID No. 3 or ITKEEASGSSVTAEEAKKFLA according to SEQ ID No. 4. Advantageously, the peptides of SEQ ID No. 3 and SEQ ID No. 4 were able to reduce MLLbcr rearrangements in leukemia cells after doxorubicin treatment without exhibiting toxic effects toward the cells or affecting the effects of the chemotherapeutic agent.

In preferred embodiments, the peptide comprises or consists of the amino acid sequence EIVVQDGITLITKEEASGSSVTAEEAKK according to SEQ ID No. 3. The peptide of SEQ ID No. 3 with a centrally positioned EEASGSS motif provided effective reduction of MLLbcr rearrangements in a wide concentration range.

In further embodiments, the peptide comprises the amino acid sequence ITKEEASGSSVTAEEAKKFLA according to SEQ ID No. 4. Also the peptide of SEQ ID No. 4 provided effective reduction of MLLbcr rearrangements. To simplify chemical synthesis or to enhance stability or solubility the peptides may be optimised. In embodiments, the peptide has the amino acid sequence ITKEEASGSSVTAEEAKKFLAG according to SEQ ID No. 5. The addition of G provides better solubility. In other embodiments, the peptide has the amino acid sequence ITKEEASGSSVTAEEAKKFLAP according to SEQ ID No. 6.

The peptides, in addition to the sequence according to any of SEQ ID No. 1 to SEQ ID No. 7 may contain additional N- and/or C-terminally located stretches of amino acids that are not necessarily part of the motif that binds to EndoG. Such additional amino acid stretches can be useful, for example, to provide an efficient introduction of the peptide into the nucleus of a cell. In embodiments, the peptide is part of a fusion protein, in particular comprising a nuclear localization signal (NLS), or a hybrid molecule for nuclear entry. The term "hybrid molecule" refers to a chemical entity with two or more structural domains having different biological functions and dual activity. A hybrid molecule may refer to a peptide comprising two peptide motifs with different function, but also may refer to a peptide-oligonucleotide hybrid molecule. A hybrid molecule for nuclear entry comprises an amino acid sequence or an oligonucleotide sequence that tag the peptide of the invention for import into the cell nucleus by nuclear transport. A nuclear localization signal may preferably be present located C-terminally. A preferred fusion protein may comprise, for example, the NLS motif of Ku80 corresponding to the amino acid sequence PTAKKLK (SEQ ID No. 15).

The peptides are usable in form of pharmaceutically acceptable salts thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines. Preferably, the pharmaceutically acceptable salt is selected from the group of alkaline and earth alkaline salts, particularly sodium or potassium salts. Also, calcium or magnesium salts can be preferred. Generally, peptides may be synthesized by standard solid-phase peptide synthesis, and reagents for peptide synthesis are commercially available.

A further aspect of the present invention relates to the peptide according to the invention for use as a medicament. For the description of the peptide, reference is made to the description above. Preferred peptides are peptides of the sequence according to any of SEQ ID No. 1 to SEQ ID No. 7 or pharmaceutically acceptable salts thereof or may be part of a fusion protein, in particular comprising a nuclear localization signal (NLS), or a hybrid molecule for nuclear entry. In preferred embodiments, a peptide EIVVQDGITLITKEEASGSSVTAEEAKK according to SEQ ID No. 3 may be used.

The peptides were found to inhibit EndoG with high specificity, but were shown to not inhibit the cytotoxic effects of chemotherapeutic agents. This enables for an additional, preventive treatment with the EndoG inhibiting peptides that can reduce the side effect of leukemia-causing MLLbcr rearrangements as desired, but will not limit the tumor cell killing intended by chemotherapy. Thus, the peptides provide the advantage that secondary leukemias, one of the most severe side effects of chemotherapy and radiotherapy, can be reduced by preventive combination therapy with the peptides.

Patients with therapy-related acute myeloid leukemia (AML) or, more rarely, acute lymphoblastic leukemia (ALL) represent a small subset of AML or ALL patients who have received prior cytotoxic therapies, including chemotherapy and/or radiotherapy for a prior malignancy, particularly for treatment of solid or hematologic cancers. Therapy-related AML or ALL also is referred to as secondary AML or ALL. *MLL* rearrangements, however, can also be caused in the foetus still in the uterus, such as when the pregnant mother is exposed to radiation. This can cause infant ALL or infant AML. Another example is bone marrow transplantation that can cause *MLL* rearrangements and secondary leukemia due to excessive self-renewal of the transplanted hematopoietic stem cells.

In preferred embodiments, the peptide is for use in the prevention of acute lymphoblastic leukemia (ALL), myelodysplastic syndrome (MDS) or acute myeloid leukemia (AML), and particularly for use in the prevention of therapy-related acute lymphoblastic leukemia, therapy-related myelodysplastic syndrome or therapy-related acute myeloid leukemia.

The peptides are able to prevent secondary leukemias when administered in addition to an active substance, such as a chemotherapeutic agent directed against a tumor. The peptides specifically reduce the secondary leukemia risk after chemotherapy, in contrast to simple dose reduction of the primary therapeutic agent, as the peptides are able to provide protection against leukemia-causing genome rearrangements without reducing the toxic effect of the therapeutic agent, e.g. on tumor cells. The peptide is highly specific for the molecular target structure EndoG and specifically protective against carcinogenic genome alterations, thus at the same time does not suppress or at least suppress the toxicities achieved by chemotherapy or radiotherapy less and thus the success of the therapy.

For use as a medicament the peptide can be used or included in a pharmaceutical composition. Accordingly, a further aspect relates to a pharmaceutical composition comprising as an active ingredient a peptide according to the invention or a pharmaceutically acceptable salt thereof, and, optionally, pharmaceutically acceptable carriers, excipients and/or vehicles. For the description of the peptides, reference is made to the description above. Preferred peptides are peptides of the sequence according to any of SEQ ID No. 1 to SEQ ID No. 7, or pharmaceutically acceptable salts thereof, or may be part of a fusion protein, in particular comprising a nuclear localization signal (NLS), or a hybrid molecule for nuclear entry. In preferred embodiments, the pharmaceutical composition comprises the peptide EIVVQDGITLITKEEASGSSVTAEEAKK according to SEQ ID No. 3.

In preferred embodiments, the pharmaceutical composition is for use in the prevention of acute lymphoblastic leukemia (ALL), myelodysplastic syndrome (MDS) or acute myeloid leukemia (AML), particularly for use in the prevention of therapy-related acute lymphoblastic leukemia (AML), therapy-related myelodysplastic syndrome (MDS) or therapy-related acute myeloid leukemia (AML).

The composition can be suitable for parenteral administration, including subcutaneous, intramuscular or intravenous administration. Compositions suitable for parenteral administration may be prepared as solutions or suspensions of the peptide in water or isotonic buffers. Compositions suitable for injectable use include sterile aqueous solutions or dispersions. The composition may be produced under sterile conditions using standard pharmaceutical techniques well known to those skilled in the art. For compositions convenient pharmaceutical media may be employed. For example, water, glycols, oils, alcohols and the like may be used to form liquid preparations such as solutions. The composition may comprise a pharmaceutical carrier, which can be, for example, a solid, liquid, or gas. Suitable carriers preferably are liquid and correspond to the substances ordinarily employed in formulation technology for pharmaceutical formulations.

The present invention also relates to the use of a peptide according to the invention, for the manufacture of a medicament, particularly for use in the preventive treatment of acute lymphoblastic leukemia, myelodysplastic syndrome or acute myeloid leukemia, preferably for use in the preventive treatment of therapy-related acute lymphoblastic leukemia, therapy-related myelodysplastic syndrome or therapy-related acute myeloid leukemia. For the description of the peptides, reference is made to the description above. Preferred peptides are peptides according to any of SEQ ID No. 1 to SEQ ID No. 7, or pharmaceutically acceptable salts thereof, or may be part of a fusion protein, in particular comprising a nuclear localization signal (NLS), or a hybrid molecule for nuclear entry. In preferred embodiments, the pharmaceutical composition comprises the peptide EIVVQDGITLITKEEASGSSVTAEEAKK according to SEQ ID No. 3.

A further aspect of the present invention relates to a method of preventing acute lymphoblastic leukemia, myelodysplastic syndrome or acute myeloid leukemia, particularly therapy-related acute lymphoblastic leukemia, therapy-related myelodysplastic syndrome or therapy-related acute myeloid leukemia, the method comprising the step of administering to a subject a therapeutically effective amount of a peptide according to the invention. The term "therapeutically effective amount" is used herein to mean an amount or dose sufficient to cause an improvement in a clinically significant condition in the subject. The subject may be a mammal, particularly a human.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: The recombination frequency in K562/MLL cells, resembling *MLL*bcr rearrangements, after Ku80ct expression and treatment with doxorubicin in Figure 1A and cell survival in Figure 1B.
- Figure 2: Figure 2A shows the recombination frequencies in K562/MLL cells after nucleofection with different concentrations of the peptide of SEQ ID No. 3 (denoted Ku3) and treatment with doxorubicin. Figure 2B shows the cell survival of K562/MLL cells after nucleofection with different concentrations of the peptide of SEQ ID No. 3 (denoted Ku3) and treatment with doxorubicin.
- Figure 3: Figure 3A shows the recombination frequencies in K562/MLL cells after nucleofection with different concentrations of the peptide of SEQ ID No. 5 (denoted Ku1) and treatment with doxorubicin. Figure 3B shows the cell survival.
- Figure 4: Cell survival of K562/MLL cells after nucleofection with different concentrations of the peptide of SEQ ID No. 9 (denoted Ku2) and treatment with doxorubicin.
- Figure 5: Cell survival of K562/MLL after nucleofection with different concentrations of the peptide of SEQ ID No. 10 (denoted Ku4) and treatment with doxorubicin.
- Figure 6: Figure 6A shows the recombination frequencies in K562/MLL cells after nucleofection with the peptide of SEQ ID No. 3 (denoted Ku3), a scrambled peptide (denoted Ku3 scrambled) or water as a control, and treatment with doxorubicin, while Figure 6B shows the cell survival. Figure 6C shows the recombination frequencies in K562/MLL cells after nucleofection with the peptide of SEQ ID No. 3, a modified peptide (denoted Ku3 mut1) or water, and treatment with doxorubicin, while Figure 6D shows the cell survival.
- Figure 7: Figure 7A shows the recombination frequencies in HeLa/MLL cells after nucleofection with different concentrations of the peptide of SEQ ID No. 3 (denoted Ku3) and treatment with doxorubicin. Figure 7B shows the cell survival. Figure 7C shows the results of genomic PCR for HeLa/MLL cells after nucleofection with the peptide of SEQ ID No. 3 (denoted Ku3) and treatment with doxorubicin.

### Materials and Methods

### Cell culture

Human K562/MLL cells, derived from K562 leukemia cells, with chromosomally integrated plasmid pHR-EGFP/3'EGFP-MLLbcr.fwd (Ireno et al. Arch Toxicol. 2014, 88(5):1141-59) were cultured in RPMI1640 medium (Gibco/Thermo Fisher Scientific, Waltham, Massachusetts, USA) and HeLa/MLL cells also with chromosomally integrated plasmid pHR-EGFP/3'EGFP-MLLbcr.fwd (Gole et al. 2015, Oncogene 34: 3391-401), were cultured in DMEM medium (Gibco/Thermo Fisher Scientific, Waltham, Massachusetts, USA) at 37°C and 5% CO₂. Both media were supplemented with 10% foetal bovine serum (Biochrom, Berlin, Germany) and 1% L-glutamine (Gibco/Thermo Fisher Scientific). To split the cultures, the HeLa/MLL cells were treated with 1x PBS (Gibco/Thermo Fisher Scientific), washed, and detached from the surface with trypsin/EDTA (PAN Biotech, Aidenbach, Germany) before a portion was transferred to the new culture flask. The cells used were free of mycoplasma according to PCR assay.

### Doxorubicin treatment

K562/MLL cells were divided 1:2 before the start of the experiment and transfected with the plasmid (2x10⁶ cells) or nucleofected with the peptide (1×10⁶ cells) on the second day. After 24 h, the cells were then treated with 2 µM doxorubicin (Uniklinik Ulm, Germany) and after 4 h incubation, the cells were centrifuged, the supernatant discarded and the cells taken up in 2 ml PBS (Gibco/Thermo Fisher Scientific). After centrifugation again, the supernatant was discarded and the cells were seeded in 2 ml of new culture medium for 72 h.

For FACS measurements, the cells were centrifuged, the supernatant removed and taken up in PBS/0.2% EDTA (USB Chemicals, Gujarat, India).

HeLa/MLL cells (0.5×10⁶) were nucleofected with the peptide on the first day and split into three 6-wells for further cultivation. On the second day, the cells were treated with 0.5 µM doxorubicin for 4 h. The medium was then removed. Afterwards, the medium was removed, the cells were washed with 2 ml PBS and 2 ml new medium was added. For FACS measurements, the cells were washed with PBS after 72 h of culture, 200 µl of trypsin (PAN Biotech, Aidenbach, Germany) was added and the reaction was stopped with medium. Genomic DNA was isolated without recultivation after doxorubicin treatment.

For the isolation of genomic DNA, the HeLa/MLL cells were seeded and nucleofected after 24 h with peptide. After another 24 h, the cells were treated with 0.5 µM doxorubicin for 4 h and fixed.

### Plasmids

The plasmids for the expression of different Ku80 variants were generated in pCMV6-AN-DDK from Origene (Rockville, Marykand, USA). Accordingly, the cDNA sequences were cloned in for wtKu80 (full length of wild-type Ku80 protein) and Ku80mut (full length of protein with mutations E720A and E721A). For Ku80ct, the NLS motif of Ku80 encoding amino acids P562-K568 (PTAKKLK) was cloned in front of the cDNA segment encoding the C-terminal Ku80 fragment V592-D709. After amplification of the plasmids in *E.coli* and purification with the PowerPrepTM HP Plasmid Maxi-Prep System (OriGene, Rockville, Maryland, USA), the plasmids were sequenced in Microsynth Seqlab (Göttingen, Germany) and stored at -20°C until use.

### Plasmid electroporation

To introduce plasmids into suspension cells (K562/MLL), they were washed in RPMI 1640 medium, no glutamine, no phenol red (Gibco/Thermo Fisher Scientific). 2×10⁶ cells in 400 µl RPMI 1640 medium, no glutamine, no phenol red, were transferred together with plasmid into 4 mm Gene Pulser^{®}/MicropulserTM electroporation cuvettes (Bio-Rad Laboratories, Hercules, California, USA). The cells were then exposed to 200 V and 1050 µF in the Gene Pulser XcellTM electroporation system (Bio-Rad Laboratories) and then seeded in 2 ml culture medium.

For the analysis of rearrangements in chromosomally integrated plasmid pHR-EGFP/3'EGFP-MLLbcr.fwd, 40 µg of plasmid DNA was electroporated into K562/MLL cells for the expression of different Ku80 variants or empty vector and an additional 10 µg of pBS (pBlueScriptII KS; Stratagene, Heidelberg, Germany).

### Peptide manufacturing

The Ku3 peptide was produced in the group of Dr. L. Ständker (Ulm Peptide Pharmaceuticals, University of Ulm). They were prepared by solid-phase peptide synthesis and purified with an HPLC-1100. Lyophilised peptides were stored at 4°C. For use, they were dissolved at 50 mg/ml in Aqua ad iniectabilia (B.Braun, Melsungen, Germany).

### Peptide nucleofection (Amaxa^{®})

The Amaxa Cell Line NucleofectorTM Kit V and the NucleofectorTM II (Lonza, Basel, Switzerland) were used for peptide transfection. The HeLa/MLL cells were first detached from the flask (see cell culture). The cells (1×10⁶ for K562/MLL and 0.5×10⁵ for HeLa/MLL) were washed with 1x PBS and taken up in 100 µl Amaxa^{®} solution, mixed with the peptide and transferred to the flask. Programme X-01 was used for K562/MLL cells and programme 1-13 for HeLa/MLL cells. After nucleofection, the K562/MLL cells were added to 2 ml medium and the HeLa/MLL cells were seeded on three 6-well plates. After 24 h, the cells were treated with doxorubicin for 4 h. For the measurement, the cells from the three 6-well plates were reunited.

### Fluorescence-activated cell sorting (FACS)

Use of the fluorescence-based assay system for recombination measurements with K562/MLL or HeLa/MLL cells with chromosomally integrated plasmid pHR-EGFP/3'EGFP-MLLbcr.fwd is described in detail in Ireno et al. 2014 and Gole et al. 2015, respectively. For the acquisition of recombination data, cells were harvested, pelleted, resuspended in PBS and then re-sedimented. The cell pellets were stored on ice and resuspended in PBS (mixed with 0.2 % EDTA) only shortly before FACS measurement. This suspension was transferred to FACS tubes and the fluorescence of the cells was detected on the FACSCalibur^{™} (BD Biosciences, San Jose, California, USA). To achieve a separation between living and dead cells, the cell clouds were plotted linearly against FSC/SSC orientation in a dot plot diagram. Depending on cell size and granularity, the living cells were distinguished from cell debris and dead cells by placing a marker (gate) around the "living cell cloud". Subsequently, the selected cell population was separated in another dot-plot diagram with FL-1/FL-2 alignment depending on their fluorescence properties (FL-1 for green fluorescence, FL-2 for orange intrinsic fluorescence). Depending on the approach, 150,000 to 1,000,000 living cells were analyzed. Recombination frequencies were determined from the proportion of green fluorescent cells in the living cell population.

### Isolation of genomic DNA

For the isolation of genomic DNA from HeLa/MLL cells, the cells were treated with 37% formaldehyde (VWR international, Randor, Pennsylvania, USA) for 10 min and a further 5 min with 1.375 M glycine (Labochem international, Heidelberg, Germany) with constant swirling. After fixation, the medium was removed and the cells were washed twice with ice-cold PBS. Finally, ice-cold PBS containing 5 µl/ml phenylmethylsulfonyl fluoride (PMSF, Sigma-Aldrich/Merck) was added to the cells and cells were scraped off. After centrifugation and removal of the supernatant, the cells were placed in 1 ml of ice-cold lysis buffer (5.0 mM PIPES, pH 8.0, Fluka, Munich, Germany; 167 mM NaCl, VWR international; 1.2 mM EDTA from USB Chemicals/Thermo Fisher Scientific, Gujarat, India; 0.01% SDS from Merck, Darmstadt, Germany; 1.1% Triton X-100 from Sigma-Aldrich/Merck) with 5 µl Protease Inhibitor Cocktail (PIC, Sigma-Aldrich/Merck) and with 5 µl PMSF and incubated on ice for 10 min. They were then broken up using a douncer (Wheaton/DWK Life Sciences, Wertheim, Germany) and centrifuged. After removal of the supernatant, the cell components were taken up in 1 ml nuclear lysis buffer (50 mM Tris-HCl, pH 8.0, Sigma-Aldrich/Merck; 10 mM EDTA; 1.0% SDS) with 5 µl PIC and with 5 µl PMSF and incubated for 10 min on ice. The lysates were sonicated at 60% continuous intensity for 5 x 15 s with 1 min pause each. Then 50 µl were taken from the sample, mixed with 150 µl water (B. Braun, Melsungen, Germany) and 10 µl 5 M NaCl and incubated at 65°C overnight. The following day, the sample was incubated with 0.5 µl RNase A (50 µg/ml; OriGene, Rockville, Maryland, USA) for 15 min at 37°C and then for 90 min at 42°C with 5 µl Proteinase K (Sigma-Aldrich/Merck). Then 200 µl of a mixture of phenol:chloroform:isoamyl alcohol (Sigma-Aldrich/Merck) were added to the suspension, mixed and the phases separated by centrifugation. The aqueous (upper) phase was taken and mixed with 20 µl 3 M Na-acetate (Sigma-Aldrich/Merck), pH 5.2 and 500 µl ethanol (-20°C) (Sigma-Aldrich/Merck). Overnight the samples were stored at -20°C. The next day, the precipitated DNA was centrifuged and washed with 500 µl of 70% ethanol (-20°C). After drying, the DNA was dissolved in 30 µl TE buffer (10 mM Tris-HCl, pH 8.0; 1 mM EDTA). The resulting DNA solution was used for PCR.

### Polymerase chain reaction (PCR)

For the analysis of DNA breakage, PCR was performed on the isolated DNA. 20 µg DNA per preparation in a final volume of 30 µl was used. For reactions with MLL(Set2) primers 1.5 mM MgCl2 was used and for reactions with Intron20 primers 1.0 mM MgCl₂. For PCR, my-Budget dNTPs and my-Budget Taq DNA polymerase (Bio-Budget, Krefeld, Germany) were used. Gel electrophoresis was performed using a 2.5% agarose gel in which DNA was stained with SYBER Save DNA gel stain (Thermo Fisher Scientific). Band intensities were quantified using the ChemiDocTM MP Imaging System (Bio-Rad Laboratories) and Image Lab 5.2.1 software (Bio-Rad Laboratories). The primers used (Biomers.net, Ulm, Germany) had the following sequences for MLL (Set2): 5'-TTGGGTGTAATCAGTTGCCTATT-3' (SEQ ID No. 11) and 5'-GGGTGATAGCTGTTTCGGCA-3' (SEQ ID No. 12) and for Intron20: 5'-GCAACACAGGGCCCTAGTTAAT-3' (SEQ ID No. 13) and 5'-AGGCAAATCAGTCACCTTTTAATCA-3' (SEQ ID No. 14).

### Statistics

All values represent the mean of at least two data points. These were plotted and analyzed using GraphPad8 (GraphPad Software, San Diego, California, USA). The P-values were calculated first with the Kruskal-Wallis and then with the Mann-Whitney-U test (unpaired, non-parametric).

### Example 1

### Determination of effect of the C-terminal domain of Ku80 on MLLbcr rearrangements

A test system for DNA recombination based on the reconstitution of the gene encoding enhanced green fluorescent protein (EGFP) as described in EP 1 399 576 A1 was used to test the influence of the C-terminal fragment of human Ku80 or shorter C-terminus derived peptides. More specifically, in this assay the quantification of MLLbcr rearrangements was investigated using fluorescence-based analysis of DNA recombination adjacent to the *MLL*bcr*.* To measure these MLLbcr rearrangements we used K562/MLL cells, i.e. K562 cells with chromosomally integrated plasmid pHR-EGFP/3'EGFP-MLLbcr.fwd. In this plasmid, the 0.4 kb long MLLbcr, which comprises the hotspot of breakpoints in secondary leukemia patients, is flanked by two differently mutated EGFP genes (HR-EGFP and 3'EGFP). Chemotherapeutic treatment causes breaks in the MLLbcr, and these induce recombination events between the mutated EGFP genes. Recombination allows wild-type EGFP to be reconstituted and expressed via a CMV promoter. The green fluorescent EGFP gene product allows to count the proportion of green fluorescent cells in the total population of living cells and to calculate the recombination frequency.

For comparative analysis of the C-terminal fragment of human Ku80, expression constructs were generated for human wild-type Ku80 (wtKu80), its C-terminal domain (Ku80ct; Ku80 amino acids 592-709 fused behind the amino acids PTAKKLK of the nuclear localization signal, NLS) and Ku80 protein, inactive with respect to non-homologous end joining (NHEJ) due to amino acid exchanges E720A and E721A at the DNA-PKcs binding site (Ku80mut). These expression constructs were introduced by electroporation into K562/MLL cells. The measurements of MLLbcr rearrangements were carried out after treatment with the chemotherapeutic drug doxorubicin, which is frequently administered to tumor patients.

Therefore, three Ku80 constructs were investigated, namely for the expression of the wild-type form wtKu80 and the variants Ku80ct and Ku80mut. Ku80ct contains an N-terminal overhang of the 7 amino acids PTAKKLK (SEQ ID No. 15), i.e. P562-K568 from the NLS (nuclear localization signal) of Ku80 followed by amino acids 592-709 from the C-terminal domain of Ku80 plus stop codon. The extreme C-terminus of Ku80 (amino acids 719-732) binds the catalytic subunit of DNA-dependent protein kinase (DNA-PKcs), allowing Ku70 and Ku80 to assemble the total DNA-PK complex. In Ku80mut, the amino acid exchanges E720A and E721A were introduced into Ku80, which prevent the interaction between Ku80 and DNA-PKcs.

K562/MLL cells were electroporated with these expression plasmids for the different Ku80 variants or the empty vector (control) and 24 h later treated for 4 h with the chemotherapeutic agent doxorubicin (2 µM). After washing the cells, they were cultured in fresh medium for a further 72 h. The subsequent detection of green fluorescent cells for analysis of recombination frequencies was achieved by FACS using a diagonal gate in a FL-1/FL-2 dot plot diagram. Living cells were pre-selected by FSC/SSC gating to distinguish between EGFP-positive and -negative within the living cells.

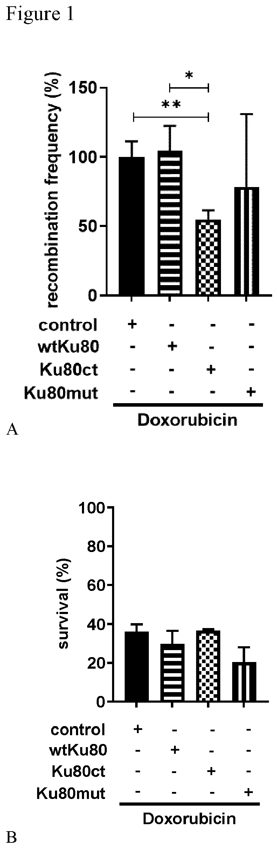

Figure 1A shows the recombination frequencies of K562/MLL cells after expression of Ku80 variants wtKu80, Ku80ct and Ku80mut, and Figure 1B illustrates the cell survival. Shown are the mean values +SEM of 6 measured values from 3 independent experiments (Mann-Whitney U test: p<0.05^{∗}; p<0.01^{∗∗}). The mean values of the controls were set to 100% for each measurement day (100%: 2x10⁵) and all other values were related to them. Cell survival according to FSC/SSC gating was between 20-40% after doxorubicin treatment.

As can be seen in Figure 1A, Ku80ct expression significantly lowered MLLbcr rearrangements by 45%, while wtKu80 had no effect and Ku80mut caused extreme fluctuations in the measurement results. To check whether cell survival was affected, it was additionally distinguished between live and dead cells by FSC/SSC gating in the FACS analyses. As expected, the toxicity of doxorubicin resulted in about 40% survival of the cells. However, as can be seen in Figure 1B, survival was not further reduced or increased by Ku80ct. This shows that no toxic or protective effects were observed after expression of wtKu80 or Ku80ct, but a statistically not significant decrease in survival was observed after expression of Ku80mut.

### Example 2

### Peptide selection and synthesis

Peptides were elected using in silico analyses, based on the sequence and 3D partial structure of human Ku80 (PDB ID 1q2z), the sequence of human EndoG (Uniprot ID Q14249), the 3D structures of the homologous EndoG/inhibitor complexes, namely EndoG/EndGI from D. melanogaster (PDB ID 3ism) and NucA/NuiA from the cyanobacterium Anabaena (PDBID 2o3b) as structural information. Sequence alignments were carried out using programs MAFFT (Katoh et al. 2002; Katoh et al. 2013), MaxAlign (Gouveia-Oliveira et al. 2007) and EMBOSScons (Rice et al. 2000) and software PPI-Detect (Romero-Molina et al. 2019). Further screening was performed using protein-peptide docking calculations with CABS-DOCK (Ciemny et al. 2016).

Starting from the sequence SEQ ID No. 3, 100,000 derivatives with a maximum of 10 modifications each were screened with PPI-Detect. Peptides that showed a score for interaction with EndoG equal to or greater than SEQ ID No. 3 were selected to build a multiple sequence alignment (MSA) and identify conserved motifs that may be responsible for the observed activity. The MAFFT, MaxAlign and EMBOSScons codes were used to align, refine, and extract conserved motifs. Consensus positions were accepted if they had positive matches (plurality) of at least half of the entries in the MSA. Subsequently, using a stricter definition of the consensus approach (i.e. a plurality of 10000 positive matches), the motif EX₁EAX₂SGSS (SEQ ID No. 1) was shown to provide the strongest rule to determine the activity of the peptide. This motif can be abstractly described as two connected fragments: 1) a fragment containing two negatively charged residues followed by at least one small and hydrophobic residue [EX₁EAX₂], and 2) a fragment containing at least four small and polar residues [X₂SGSS]. Furthermore, the screening revealed peptides among which the sequence EEASGSS was the most conserved and thus represented a potential binding motif.

The following peptides were selected based on human Ku80: ITKEEASGSSVTAEEAKKFLAP according to SEQ ID No. 6, RVLVKQKKASFEEASNQLINHIEQFL according to SEQ ID No. 9, also denoted "Ku2", EIVVQDGITLITKEEASGSSVTAEEAKK according to SEQ ID No. 3, also denoted "Ku3", and ASFEEASNQLINHIEQFLDTNETPYFMKSI according to SEQ ID No. 10, also denoted "Ku4".

The in silico designed peptides were produced synthetically. Regarding the peptide of SEQ ID No. 6 the C-terminal proline was replaced with a foreign glycine (-G) for the purpose of efficient peptide synthesis and purification. Accordingly, peptide ITKEEASGSSVTAEEAKKFLAG according to SEQ ID No. 5, also denoted "Kul", was used.

### Example 3

### Determination of the effects of peptides of SEQ ID Nos. 3, 5, 9, and 10 on MLLbcr rearrangements

The peptides were introduced into K562/MLL cells by Nucleofector^{™} technology as described above. The cells were nucleofected with 0-600 µg peptide per 1×10⁶ cells and treated with 2 µM doxorubicin for 4 h after 24 h. After washing, the cells were cultured for another 72 h in fresh medium. After washing the cells, they were cultured for another 72 h in fresh medium. Subsequently, recombination frequencies were determined.

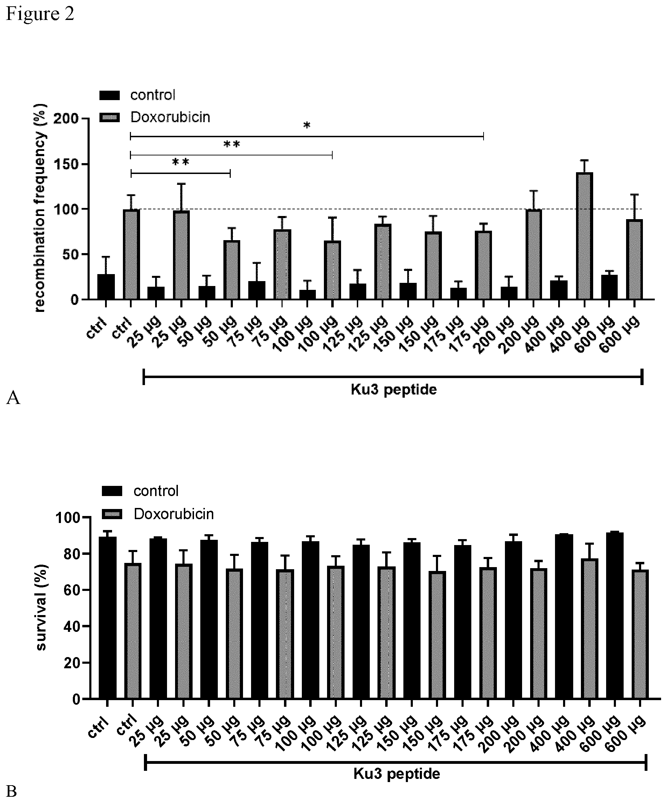

For the peptide of SEQ ID No. 3 (denoted Ku3), Figure 2A shows the recombination frequencies in K562/MLL cells. Shown are means of 3-6 individual values +SD for 0-200 µg peptide (Mann-Whitney U test: p<0.05^{∗}; p<0.01^{∗∗}); n=2 for 400 µg and 600 µg peptide. As can be seen in Figure 2A, the peptide of SEQ ID No. 3 containing the EEASGSS motif in the middle of the peptide sequence was found to reproducibly reduce MLLbcr rearrangements by up to 35% and over a wide concentration range (50 µg, 100 µg, 175 µg) after treatment with the chemotherapeutic drug doxorubicin.

Cell survival was determined in parallel with the recombination measurements. Figure 2B shows the survival of the K562/MLL cells. As can be seen in Figure 2B, the survival of 70-80% of the cells after doxorubicin treatment by nucleofection was not affected by the treatment with the peptide of SEQ ID No. 3. In untreated cells, the peptide had no significant effect.

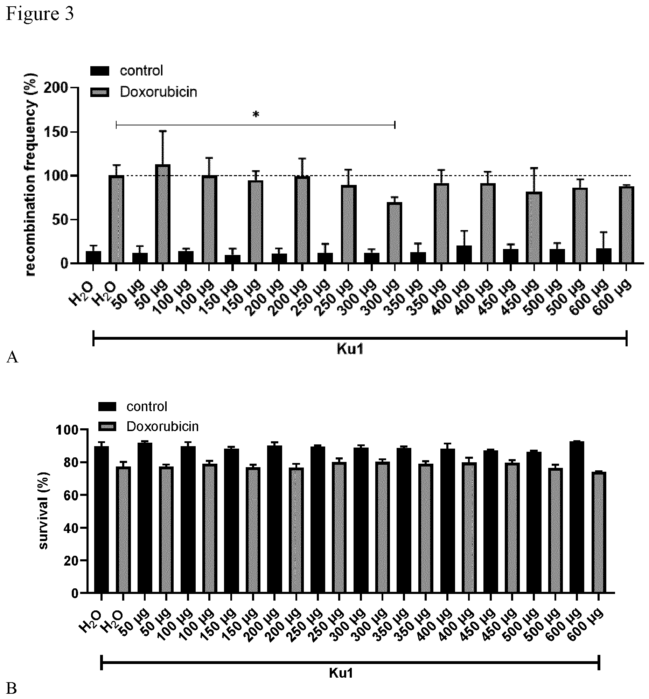

For the peptide of SEQ ID No. 5 (denoted Ku1), Figure 3A shows the recombination frequencies in K562/MLL cells. Shown are mean values from 4-6 individual values +SD for H₂O and 100-500 µg peptide (Mann-Whitney U test: p<0.05^{∗}); n=2 for 50 µg and 600 µg peptide. Figure 3B shows the survival of the K562/MLL cells for the peptide Ku1. As can be seen in Figures 3A and 3B, the peptide of SEQ ID No. 5 showed significant inhibition of MLLbcr rearrangements with a 30% decrease after application of only one of the tested peptide concentrations (300 µg), and also did not affect cell survival.

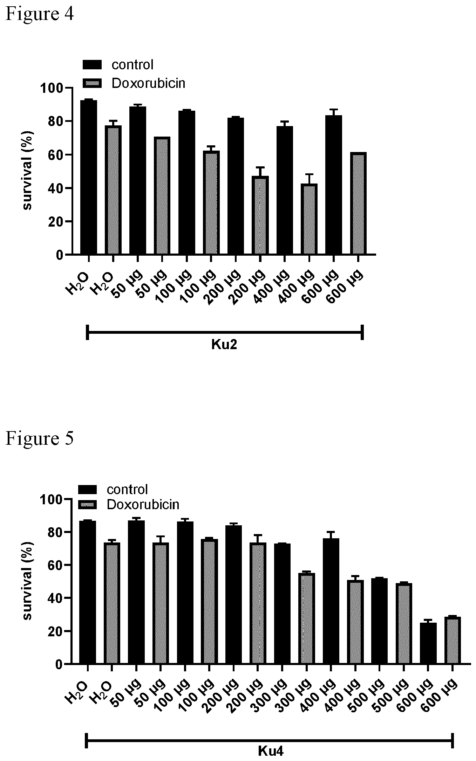

Figure 4 shows the survival of the K562/MLL cells for the peptide of SEQ ID No. 9 (denoted Ku2). Figure 5 shows the survival of the K562/MLL cells for the peptide of SEQ ID No. 10 (denoted Ku4). As can be taken from Figures 4 and 5, in contrast to the peptides of SEQ ID No. 3 and SEQ ID No. 5, the peptides RVLVKQKKASFEEASNQLINHIEQFL (SEQ ID No. 9) and ASFEEASNQLINHIEQFLDTNETPYFMKSI (SEQ ID No. 10) caused toxicities. Further, a reduction of cell survival after doxorubicin treatment to ≥ half after nucleofection with 200-400 µg of the peptide of SEQ ID No. 9 (Ku2) or ≥300 µg of the peptide of SEQ ID No. 10 (Ku4) was observed.

Survival of K562/MLL cells was cross-checked using either a scrambled peptide VDSTTIEKSIAEVAEEQESILKKVAGGT (SEQ ID No. 8), in which the amino acids contained in the peptide of SEQ ID No. 3 were randomly mixed, or a modified peptide EIVVQDGITLITKAEASGSSVTAEEAKK (SEQ ID No. 16), in which the first E of the EEASGSS motif was replaced by an alanine. The peptide of SEQ ID No. 3 and the scrambled or modified peptides were introduced into K562/MLL cells by nucleofection as described above. As a control, cells were nucleofected with H₂O only. After 24 h, cells were treated with 2 µM doxorubicin for 4 h and then washed with PBS and seeded in new medium. After a further 72 h, recombination frequencies were quantified as described above and the cell survival was determined.

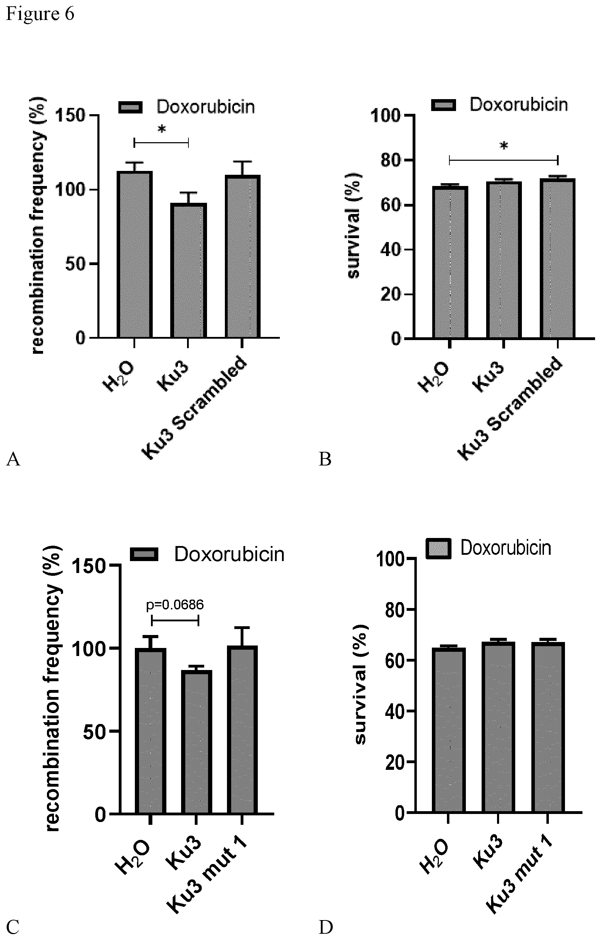

Figure 6A shows the recombination frequencies and Figure 6B shows the cell survival for the scrambled peptide. Shown are mean values +SEM (Mann-Whitney U test: p<0.05^{∗}) for H₂O (n=18) and 100 µg peptide (n=9). As can be seen in Figures 6A and 6B, while 100 µg of the peptide with SEQ ID No. 3 (denoted Ku3) significantly reduced the recombination frequency compared to water, no reduction was observed with 100 µg scrambled peptide, whereby survival was not affected for either peptide (Figure 6B). This verifies that the peptide with SEQ ID No. 3 (denoted Ku3) affects MLLbcr rearrangements in a sequence-specific manner.

Figure 6C shows the recombination frequencies and Figure 6D shows the cell survival for the modified peptide. Shown are mean values +SEM (Mann-Whitney U test: p<0.05^{∗}) for the data (n=12-15, from 5 individual experiments). Normalization was performed to H₂O 100% with a recombination frequency of 9×10⁵. Outliers of 5% were excluded following the ROUT calculations.As can be seen in Figure 6C, 50 µg of the peptide with SEQ ID No. 3 decreased the recombination frequency with a trend to statistical significance (p<0.1) compared to water, while no reduction was observed with 50 µg of the modified peptide (denoted Ku3 mut1). That only a trend (p<0.1) was observed for the reduction of MLLbcr rearrangements is assumed to be due to the fact that only 50 µg of the peptides were used for nucleofection as compared to 100 µg in the control experiments using scrambled peptide. Cell survival was not affected for either peptide as can be seen in Figure 6D.

These results show that in contrast to the peptides comprising the motif EEASGSS, the peptides of SEQ ID No. 9 and 10, which did not comprise the complete EEASGSS motif, caused toxicities and further reduced cell survival after doxorubicin treatment, while a scrambled peptide or a modified peptide neither reduced the recombination nor affected survival. It was assumed that the motif is crucial for specific EndoG inhibition without affecting anti-tumor treatment.

### Example 4

### Determination of the effect of the peptide of SEQ ID No. 3 in different cell types

To verify whether the inhibitory effect of the peptide of SEQ ID No. 3 can be observed independently of cell type, HeLa/MLL cells were analyzed after nucleofection with Ku3 peptide at different concentrations using Nucleofector^{™} technology. Cells were nucleofected with 0-100 µg peptide of SEQ ID No. 3 per 0.5×10⁶ cells. After 24 h of cultivation, the cells were treated with 0.5 µM doxorubicin for 4 h. The cells were then washed. After washing, the cells were transferred to fresh medium and cultured for another 72 h. Subsequently, recombination frequencies were determined as described above for K562/MLL. Cell survival was determined in parallel with the recombination measurements.

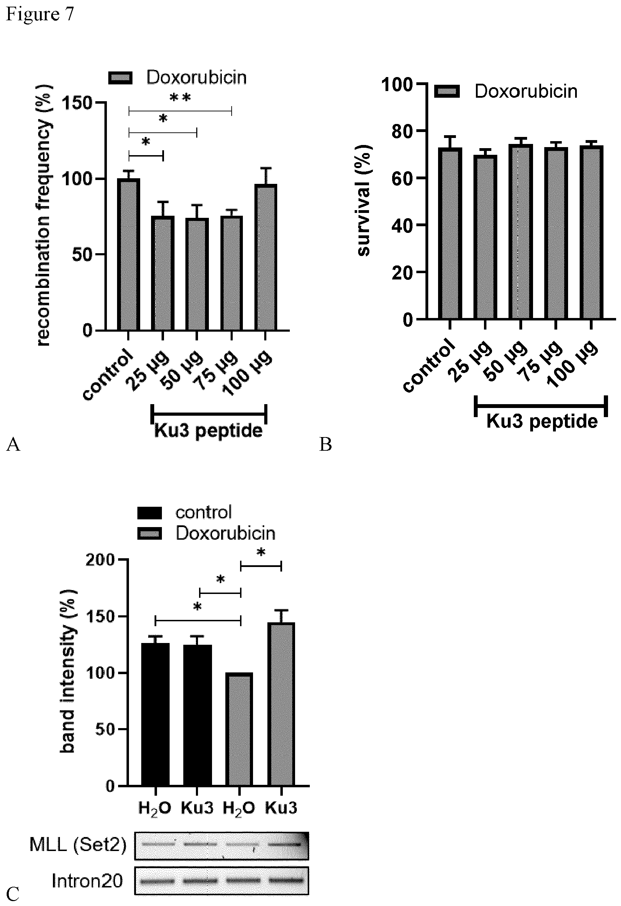

The Figure 7A shows the recombination frequencies in HeLa/MLL cells. Means of 7-10 individual values +SD are shown (5% outliers were excluded using the ROUT method; Mann-Whitney U test: p<0.05^{∗}; p<0.01^{∗∗}). Mean values of controls were set to 100% for each day of measurement (100%: 1x10⁴) and all other values were referred to it each. The Figure 7B shows the cell survival of HeLa/MLL cells. As can be taken from Figure 7A and 7B, there was a significant reduction of MLLbcr rearrangements by up to 26% in the range of 25 µg to 75 µg after treatment with doxorubicin without affecting cell survival (Figure 7B). At the higher concentration of the peptide (denoted Ku3) of 100 µg, the inhibitory effect was lost in HeLa/MLL cells as well as for ≥200 µg in K562/MLL cells, which could be explained by residual impurities after peptide synthesis.

Furthermore, genomic PCR was performed. For this, HeLa/MLL cells were nucleofected with 75 µg Ku3 per 0.5×10⁶ cells and treated with 0.5 µM doxorubicin for 4 h after 24 h. The genomic DNA was then isolated and PCR for MLL (Set2) and Intron20 performed followed by gel electrophoresis and image analysis of the band intensities. The results of the corrected and averaged band intensities from seven experiments are shown in Figure 7C (mean +SEM; Wilcoxon test: p<0.05^{∗}). As can be taken from Figure 7C, the nucleofection of HeLa/MLL cells with the peptide of SEQ ID No. 3 (denoted Ku3) protected MLLbcr from doxorubicin-induced breakage.

## Claims

1. A peptide comprising an amino acid sequence EX₁EAX₂SGSS according to SEQ ID No. 1, or a pharmaceutically acceptable salt thereof, wherein X₁ and X₂ independently represent a linker which links the adjoining amino acids or an amino acid deletion, and wherein the peptide has an overall length in the range of from 7 to 30 amino acids.

2. The peptide according to claim 1, wherein X₁ and X₂ independently represent one or two contiguous amino acids selected from the group of glutamic acid, serine, alanine, glycine or an amino acid deletion.

3. The peptide according to claim 1 or 2, wherein the amino acid sequence is EEASGSS according to SEQ ID No. 2.

4. The peptide according to any one of the foregoing claims, wherein the peptide has an overall length in the range of from 20 to 28 amino acids.

5. The peptide according to any one of the foregoing claims, wherein the peptide comprises or consists of the amino acid sequence EIVVQDGITLITKEEASGSSVTAEEAKK according to SEQ ID No. 3 or ITKEEASGSSVTAEEAKKFLA according to SEQ ID No. 4.

6. The peptide according to any one of the foregoing claims, wherein the peptide is part of a fusion protein, in particular comprising a nuclear localization signal or a hybrid molecule for nuclear entry.

7. The peptide according to any one of claims 1 to 6, for use as a medicament.

8. The peptide for use according to claim 7, wherein the peptide is for use in the prevention of acute lymphoblastic leukemia (ALL), myelodysplastic syndrome (MDS) or acute myeloid leukemia, particularly for use in the prevention of therapy-related acute lymphoblastic leukemia, therapy-related myelodysplastic syndrome (MDS) or therapy-related acute myeloid leukemia.

9. A pharmaceutical composition comprising as an active ingredient a peptide according to any one of claims 1 to 6 or a pharmaceutically acceptable salt thereof, and, optionally, pharmaceutically acceptable carriers, excipients and/or vehicles.

10. The pharmaceutical composition according to claim 9, for use in the prevention of acute lymphoblastic leukemia, myelodysplastic syndrome or acute myeloid leukemia, particularly for use in the prevention of therapy-related acute lymphoblastic leukemia, therapy-related myelodysplastic syndrome or therapy-related acute myeloid leukemia.

11. The pharmaceutical composition according to any one of claims 9 or 10, wherein the pharmaceutical composition comprises the peptide EIVVQDGITLITKEEASGSSVTAEEAKK according to SEQ ID No. 3.

12. Use of a peptide according to any one of claims 1 to 6, for the manufacture of a medicament, particularly for use in the preventive treatment of acute lymphoblastic leukemia, myelodysplastic syndrome or acute myeloid leukemia, preferably for use in the preventive treatment of therapy-related acute lymphoblastic leukemia, therapy-related myelodysplastic syndrome or therapy-related acute myeloid leukemia.

13. A method of preventing acute lymphoblastic leukemia, myelodysplastic syndrome or acute myeloid leukemia, particularly therapy-related acute lymphoblastic leukemia, therapy-related myelodysplastic syndrome or therapy-related acute myeloid leukemia, the method comprising the step of administering to a subject a therapeutically effective amount of a peptide according to anyone of the claims 1 to 6.
